# EUROPEAN PATENT SPECIFICATION

(11) **EP 3 813 071 B1**
(45) Date of publication and mention of the grant of the patent: **07.08.2024**
(21) Application number: 20202942.7
(22) Date of filing: 21.10.2020
(51) Int. Cl.: G16B 40/20, G16H 50/20

(54) **METHOD FOR CREATING A COHERENT VOTING NETWORK USEFUL IN PREDICTING A LIKELIHOOD OF LONG-TERM SURVIVAL OF A BREAST CANCER PATIENT**
VERFAHREN ZUR ERZEUGUNG EINES KOHÄRENTEN ABSTIMMUNGSNETZWERKS ZUR VORHERSAGE DER WAHRSCHEINLICHKEIT DES LANGFRISTIGEN ÜBERLEBENS VON BRUSTKREBSPATIENTEN
PROCÉDÉ DE CRÉATION D'UN RÉSEAU DE VOTE COHÉRENT UTILE POUR PRÉDIRE LA PROBABILITÉ DE SURVIE À LONG TERME D'UNE PATIENTE ATTEINTE D'UN CANCER DU SEIN

(30) Priority: 22.10.2019 IT 201900019571
(43) Date of publication of application: 28.04.2021
(73) Proprietor: Consiglio Nazionale Delle Ricerche, 00185 Roma (IT)
(72) Inventor: PELLEGRINI, Marco, 56124 PISA (IT)
(74) Representative: Lavoix

(56) References cited:
- WO-A2-2006/113747
- RUNYU JING ET AL: "Ensemble Methods with Voting Protocols Exhibit Superior Performance for Predicting Cancer Clinical Endpoints and Providing More Complete Coverage of Disease-Related Genes", INTERNATIONAL JOURNAL OF GENOMICS, vol. 2018, 1 January 2018 (2018-01-01), pages 1 - 14, XP055703712, ISSN: 2314-436X, DOI: 10.1155/2018/8124950
- LI HE ET AL: "Identifying the Gene Signatures from Gene-Pathway Bipartite Network Guarantees the Robust Model Performance on Predicting the Cancer Prognosis", BIOMED RESEARCH INTERNATIONAL, vol. 2014, 1 January 2014 (2014-01-01), pages 1 - 10, XP055703723, ISSN: 2314-6133, DOI: 10.1155/2014/424509
- AMRIT SINGH ET AL: "Abstract", BIORXIV, 20 March 2018 (2018-03-20), XP055703706, Retrieved from the Internet <URL:https://www.biorxiv.org/content/10.1101/067611v2.full.pdf> [retrieved on 20200611], DOI: 10.1101/067611

## Description

The present invention relates to a method for creating a coherent voting network useful in predicting a likelihood of long-term survival of breast cancer in a breast cancer patient.

In particular, the present invention relates to a method for the elaboration of the expression levels of a panel of genes in a tumor tissue to obtain a coherent voting network that can be used for predicting a patient's survival five years after a tumor removal.

Breast Cancer is one of the main causes of death in Europe, USA and China. The number of new cases each year in Europe is 92.2 women every 100,000 women. The mortality rate in Europe is of 23.1 women every 100,000.

For a patient affected by breast cancer, after tumor removal, it is necessary to decide a therapy able to prevent the tumor relapse and the formation of metastases. To this effect, a series of measurements of several parameters (clinical, histological, molecular) are usually collected in known art methods, which are based on the use of molecular biomarkers and which are considered valid clinical decision support tools complementing traditional histopathology.

Economic forecasts predict a market of 8B USD in Europe, in 2022, for breast cancer tests (of all types, including diagnostic, prognostic and imaging). The market for breast cancer tests in the USA will be of 18 B USD in 2025. Limited to molecular testing (for all types of cancer), the Chinese market will be worth 1.5 B USD in 2022.

Two of the most used molecular prognostic tests are Mammaprint ^{®} (product of Agendia) and Oncotype DX ^{®} (product of Genomic Health, Inc). At this moment in the USA a Mammaprint ^{®} test has a cost of 4.200 USD, while in Europe the cost is 2.675 Eur. Onotype DX ^{®} test has a cost in the USA of 3.416 USD. Prognostic molecular tests are cost-effective versus the cost of chemotherapy for patients who would not eventually benefit from it. They are considered complementary to histology-based more traditional methods (e.g. TNM staging).

The main disadvantage of the two tests above indicated is the fact that too many false positive are selected. Runyu, J. et al,International Journal of Genomics, vol. 2018, p.1-14 discloses the combination of multiple existing predictive models for predicting cancer clinical endpoints with voting protocol. Li, H et al BioMed Research International, vol 2014, p1-10, describes a method for predicting cancer prognosis based on gene pathway bipartite networks identifying the most important pathways in cancer based on gene signatures.

There is therefore the need to develop a method for creating a coherent voting network useful in predicting a likelihood of long-term survival of a breast cancer patient which is more efficient and which allows reducing the number of false positive, thus overcoming the limitations of the prior art.

The main technical problem solved by such a method is how to select, among a possible number of about 25,000 molecular biomarkers (mRNA) expressed in a biopsy of a tumor tissue, a reduced panel of biomarkers (few dozen mRNA) that can be measured at a reduced cost, and that can help predicting survival after five years, in dependence of a chosen post-operative therapy.

These and other objects are fully achieved by virtue of a method for creating a coherent voting network useful in predicting a likelihood of long-term survival of breast cancer in a breast cancer patient having the characteristics defined in independent claim 1.

Preferred embodiments of the invention are specified in the dependent claims, whose subjectmatter is to be understood as forming an integral part of the present description.

Further characteristics and advantages of the present invention will become apparent from the following description, provided merely by way of a non-limiting example, with reference to the enclosed drawings, in which:
- Figure 1 is a block diagram of the steps of a method for creating a coherent voting network according to the present invention; and
- Figure 2 is a block diagram of the steps of a method for selecting a panel of biomarkers and a parameter vector useful in predicting a likelihood of long-term survival of breast cancer in a breast cancer patient which uses a coherent voting network according to the present invention.

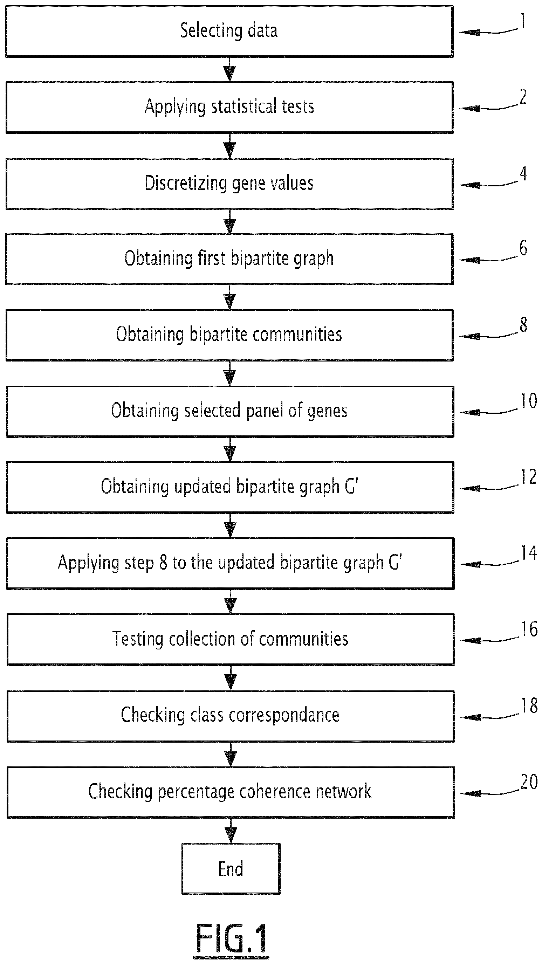

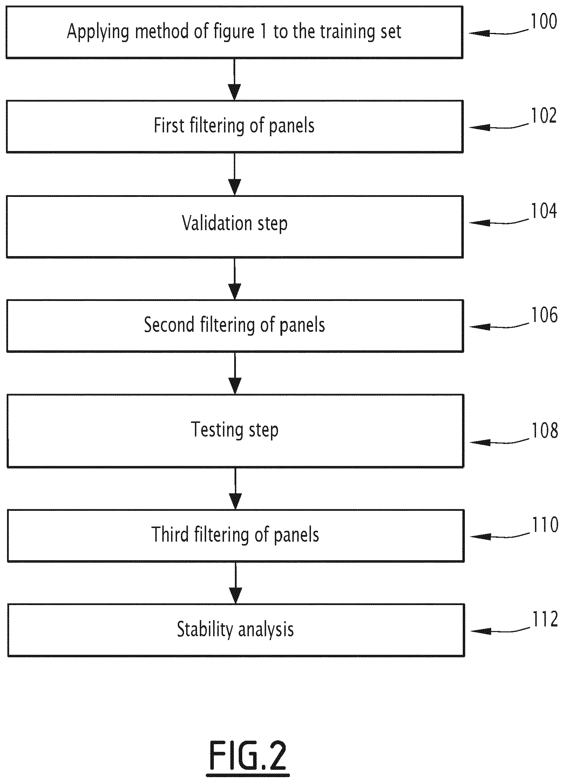

In the following of the description, the term "*genes"* will be used when referring to intermediate steps of the methods of figures 1 and 2, and "*biomarker*" will be used when referring to the final panel of genes selected according to the method of figure 2.

The method of the present invention can be carried out by a system comprising, in a manner per se known, a workstation having an elaboration unit, a display device and a network bus for connecting to a data network.

The workstation is arranged to elaborate modules and programs stored on a storage memory or accessible through the network data, to display the results on the display device.

### 1. Construction of a coherent voting network.

Figure 1 is a block diagram of the steps of a method for creating a coherent voting network (also called predictor) according to the present invention.

Each step in Figure 1 depends on input from previous steps and on some algorithmic parameters that a user must set, within a given range of possible values (the precise parameters will be specified in the following of the description).

The collection of values of such input parameters is called *"parameter vector",* and the collections of all parameter vectors is called *"parameter space".*

In an initialization phase, the method of Figure 1 uses data publicly available taken from the Molecular Taxonomy of Breast Cancer International Consortium METABRIC. METABRIC is a cohort of about 2000 patients with full molecular (mRNA) and clinical data associated to, including post-surgery treatments and survival data indicating whether the patient has actually survived or not five years after the tumor removal.

At step 1, data from the cohort of 2000 patients available through the METABRIC consortium are selected and organized in a master matrix having in its row the list of patients, each having an associated survival or not-survival class A or B (the survival data) indicating whether the patient had survived or not five year after the tumor removal, and having in its column the expression value of a panel of genes including about 24.000 genes.

At step 2, a known statistical test, such as t-test, Kolmogorov-Smirnov and Mann-Whitney U test, are applied to each gene of the master matrix in order to evaluate which genes better discriminates the A and B classes.

Step 2 requires setting the value of a first group of the parameters of a parameter vector including the type of test performed, the maximum p-value for accepting a gene among those under test, and a threshold for accepting a fold change of the gene under test. These first parameters are per se known.

Step 2 allows reducing the initial 24.000 genes to a reduced number of genes, the so-called first candidate panel of genes, preferably in the range of 500-1000 genes. This first candidate panel is still too high, therefore, a further reduction will be performed in step 10 as described here below.

At step 4, the expression value of each of the genes belonging to the first candidate panel is discretized in sub-intervals capable of discriminating the A and B classes, by applying known methods based on the information theory.

Step 4 requires setting the value of a second group of the parameters of the parameter vector including the specific objective function used for the determination of cut points, the minimum and maximum number of cut points generated, the minimum and maximum number of patients (in percentage) in each interval generated by a cut point, and the number of significant digits to be considered in the gene expression measurements. These second parameters are per se known.

At the end of step 4, in the master matrix, each expression value of the genes belonging to the first candidate panel is replaced by an interval.

At step 6, the quantized master matrix built at step 4 is converted in a first bipartite graph G comprising patient-nodes of both classes A, B, and gene-nodes representatives of the sub-intervals of the expression value of genes of the first candidate panel. In order to obtain this bipartite graph G, all the rows of the master matrix are used while only the columns of the master matrix whose values of the genes are the ones discretized are used; the other columns are discarded.

The transformation of step 6 is useful for the application of graph-theoretic methods, and moreover, it removes the need of treating values missing in the original matrix, wherein a missing value in the matrix is just a missing edge in the bipartite graph.

At step 8, a predetermined algorithm, for example the "Core & peel" algorithm, is applied to the bipartite graph G, this algorithm providing in output a collection of bipartite communities including both patient-nodes and gene-nodes, each community being dense and the collection of communities providing high coverage of the nodes of the bipartite graph G.

Step 8 requires setting a third parameter, a density threshold, i.e. the percentage of required edges over the maximum possible number of edges of the bipartite complete graph with the same nodes. Typical density threshold values are 0.6, 0.7, 0.8 and 0.9.

At step 10, a further predetermined algorithm, for example the greedy-set-multi-cover algorithm, is applied to the communities obtained at the end of step 8, restricted to the gene-nodes only.

This algorithm produces in output a subset of the genes of the first candidate panel. This subset of genes is typically of much smaller size, preferably 15 genes. This subset of genes is a second candidate panel and its ability to reproduce the same structure of bipartite communities obtained at step 8 is tested in the following steps.

Step 10 requires setting a fourth parameter, a covering number for the greedy-set-multi-cover algorithm, which is per se known and is typically chosen between 3 and 7.

At step 12, an updated bipartite graph G' is created comprising nodes-patients and nodes-gene, whose genes belongs to the second candidate panel generated at step 10.

At step 14, step 8 is repeated on the updated bipartite graph G' and a check is performed to verify that the communities obtained at this step are similar to the ones obtained at step 8.

In negative case, the method ends and advantageously an alert can be displayed on the display device.

At step 16, the communities obtained at step 14 are considered only for the patient-nodes. The collection of patients of the patient-nodes represents a voting network, and a further test to determine if it is coherent or incoherent is performed in the following steps.

In particular, each community applies a decision function on each patient belonging to it to determine whether to assign her at the A or B class. Then, for each patient, it is checked to which community she belongs to, which vote each of said communities has assigned to her, and finally a final class is assigned to the patient based on a majority rule, i.e., the patient is assigned the class A o B which has received the majority of vote from the communities she belongs to.

Step 16 requires setting a fifth parameter, a decision function among a predetermined set of functions. An example of function is "unanimity" (patient p in community c is assigned to class A if all patients in community c, except p, are of class A; patient p in community c is assigned to class B if all patients in community c, except p, are of class B; in all other cases no class is assigned to p). A second example of such functions is "majority"' (patient p in community c is assigned to class A if more patients in community c, except p, are of class A rather than B; patient p in community c is assigned to class B if more patients in c, except p, are of class B rather than A; if there is a tie among the patients in c, excluded p, no class is assigned to p).

Further decision functions are based on the result of an hypergeometric test on the distribution of lables (A and B) of patients of a community with respect to the larger set of patients being processed.

At step 18, for each patient belonging to the various communities of step 16, it is checked whether the class assigned at step 16 is the same as the class stored in the METABRIC database referring to said patient. If the class is coincident, the patient is declared "coherent", otherwise "incoherent".

At step 20, it is checked whether the percentage of coherent patients in the voting network is greater than a predetermined threshold, for example 90%, and, in positive case, the network is called coherent and can be further used to classify any new unknown patient by adding her to the set of patients.

The above description discloses the method of Figure 1 in an initialization phase wherein the master matrix has been construed by using the data of all the 2000 patients of the METBRIC consortium.

In order to perform a method for selecting a final panel of biomarkers and a final parameter vector according to the disclosure of Figure 2, the method for creating a coherent voting network of figure 1 is in turn applied to subset of the 2000 patients, as here below detailed.

In particular, the method of Figure 2 includes a training phase, a validation phase, a testing phase and a stability analysis phase, and the cohort of about 2000 patients of the METABRIC consortium is split randomly into about 1000 patients for the training phase (training set), about 500 patients for the validation phase (validation set), and about 500 patients for the testing phase (testing set).

Therefore, these subsets of patients (training set, validation set and testing set) are used to create sub-matrices, starting from the master matrix, to be processed separately.

Figure 2 is a block diagram of the steps of a method for selecting a panel of biomarkers and a parameter vector, which performs a *"model selection",* i.e. this method, from input data, produces a single configuration (a final panel of biomarkers and a final parameter vector) that uniquely defines a coherent voting network with good predictive properties.

Figure 2 shows how to select the best coherent voting network among all the coherent voting networks that can be built by using the method of figure 1, by modulating the first to fifth parameters above disclosed.

In the following of the description, "coherent voting network scheme" will refer to the general steps for obtaining a coherent voting network, and "coherent voting network" will refer to a specific coherent voting network obtained by running the scheme with specific input parameters.

The method of Figure 2 has two objectives: (a) from a subset of initial 24,000 genes, it allows finding a small panel of genes (the final panel of biomarkers) with good predictive properties, for some parameter vectors, and (b) it selects the final parameter vector in the parameter space that, together with the final panel of biomarkers obtained at step (a), produces a coherent voting network with good predictive properties.

Figure 2 represents a computational pipeline that solves the so called *"model selection problem"* for proposed coherent voting networks.

The method of Figure 2 comprises four main steps (referred as 100 - training step, 104 - validation step, 108 - testing step and 112 - stability analysis step), alternated with filtering/selecting steps (referred as 102, 106, and 110).

The main steps 100, 104, 108 and 112 will be disclosed in more detail in the following.

The filtering/selecting steps 102, 106 and 110 take in input at once all the predictors computed in the respective preceding main step 100, 104, 108, along with predetermined measures of performance (for example percentage of coherence, slackness (i.e. percentage of no responses), accuracy, and odds ratio), and filter and rank such predictors and the corresponding configurations (gene panel, parameter vector).

A predetermined maximum number of configurations corresponding to high performance predictors are then used to seed each time the subsequent steps of the pipeline of Figure 2.

More specifically, in a first filtering step 102, the predetermined measures of performances include:
- evaluating, in a manner per se known, if candidate gene panels are larger than a desired value, and discard them;
- evaluating if voting networks have a percentage of coherence below a predetermined threshold, for example 85% of the maximum coherence of all generated voting networks, and discard them as well. After ranking the coherent voting networks by percentage of coherence, a predetermined amount of corresponding gene panels are retained, for example 30 gene panels, as detailed here below.

The first filtering step 102 allows restricting only the number of gene panels to pass on to the next phase.

In a second filtering step 106, the predetermined measures of performances include:
- removing predictors that have slackness above a predefined threshold (for example 10%);
- calculating, for each predictor, a point in a two-dimensional space (Accuracy, Odds Ratio), and calculating the *"Pareto front"* of such two-dimensional set of points. The gene panels and the parameter vectors corresponding to the *"Pareto front"* are then passed to the next step.

In the second filtering step 106 both the number of gene panels and the number of parameter vectors associated to each gene panel are restricted.

In a third filtering step 110, the predetermined measures of performances include removing predictors that have slackness above a predefined threshold (for example 10%).

After that, the predictors are ranked by their odds ratio value and the configuration (gene panel and parameter vector) with the highest odd ratio value is selected.

This determines a final selected gene panel (the final panel of biomarkers) and a final parameter vector.

With this final panel of biomarkers and final parameter vector it is possible to construe a unique coherent voting network, by applying the method of Figure 1.

This final coherent voting network can be therefore used to classify any new patient, as disclosed here below.

### 2. Training, validating, testing and stability analysis of a coherent voting network

In the following, firstly, a brief overall overview of the method for determining a final panel of biomarkers and final parameter vector of the present invention will be provided, then, a detailed description of the steps involved will be presented.

The method of the present invention allows obtaining a final panel of biomarkers and a final parameter vector, which can be used to predict the survival of a breast cancer patient five years after tumor removal.

The final panel of biomarkers is obtained by measuring a level of expression of genes in a cancer breast tissue taken from a patient through biopsy.

The method is of the type *"supervised learning"*: a system undergoes a training phase using full molecular profiles and survival data of a cohort of patients per se known. The so trained system is then used with new patients, for which only a restricted panel of biomarkers is actually measured, in order to predict the 5-years prognosis, i.e. if the patient will survive or not five years after the tumor removal.

In an alternative embodiment of the invention, the training set, the validation set and the testing set above cited are each divided into a predetermined number of sub-groups including an equal number of patients, preferably eight sub-groups, depending on classes of therapies associated to each patient in the METABRIC database.

This is done because the about 2000 patients of the METABIC database are very heterogeneous, therefore, eight sub-groups corresponding to radiotherapy, chemotherapy, and hormonal therapy are treated separately.

In fact, patients after surgery may or may not follow one of the following therapies: chemotherapy, radiation therapy or hormone therapy. There are eight possible combinations of three therapies, and for each combination the training, validation and testing phases are repeated. Thus, eight therapy-specific biomarkers sub-panels can be obtained (primary stratification).

Within each therapy class the predictive quality of the panel of biomarkers is measured by using the "slackness" (S), "accuracy" (Acc), the "odds ratio" (OR), the "positive predictive value" (PPV) and the "negative predictive value" (NPV) measurements, which are per se known.

Starting from the eight sub-groups based on the therapy-classes (primary stratification), it is also possible to further define stratifications based on different features (secondary stratification), for example the hormonal classes ER+, ER-/HER2+ and TNBC.

The secondary stratification does not change the prediction of a single patient but provide a different evaluation of the quality of the prediction. A clinician thus can have diverse point of views depending on the most prominent features of the patient, judging on those prevalent for the single patient.

In the following, the method will be disclosed by referring to a single cohort of 1000 patients for the training set, 500 patients for the validation set and 500 patients for the testing set, but it can be equally applied to the eight sub-groups.

### 2.1 Training phase

In the training step 100 of Figure 2, the steps of the method of Figure 1 (the coherent voting network scheme) are applied, for a predetermined parameter vector of a predefined parameter space, to the training set.

A predetermined parameter vector includes parameters chosen among the parameters of the first to fifth group as above disclosed. A different parameter vector can include a different selection of these parameters.

The training phase has two sub-phases.

In the first sub-phase, (a) a bipartite graph is built as above disclosed, in a manner per se known starting from the data of the 1000 training patients, with nodes representing patients and nodes representing gene expression levels for each patient (of about 24.000 genes stored in the METABRIC database and associated to each patient).

Then (b) a collection of node communities with high density of connections is found in this bipartite graph in a manner per se known, each community including both nodes representing patients (patient-nodes) and nodes representing gene expression levels (gene-nodes).

Finally (c), a two level voting scheme is applied. Each community expresses a vote, survival or not survival, for each patient belonging to the community, this vote indicating whether the patient according to this community will survive five years after the tumor removal or not. Each patient collects the votes of all the communities she belongs to.

In the end, each patient is assigned to a class (survival or not survival) depending on the majority of votes she has received from each community she belongs to.

If no vote is given, or if there is a tie, no prediction is made.

In the end, the class assigned to each patient is compared with the actual survival data corresponding to the patient and stored in the METABRIC database.

It has been found that the number of patients which has been correctly classified is above a predetermined threshold, preferably 90%, i.e. the class assigned at the end of the first sub-phase of the training phase actually corresponds to the survival data of the METABRIC database, this meaning that the method has correctly operated.

The method has been therefore further developed with the next steps here below disclosed, aiming at reducing the number of genes, which are needed to define the voting communities.

In the second sub-phase, the number of genes is reduced as detailed here below, while preserving the overall scheme above disclosed, thus minimizing the number of genes whose expression is needed to complete the class assignment.

These genes form the candidate panel of genes corresponding the predetermined parameter vector, such candidate panel of genes corresponding to the second candidate panel of genes above disclosed with reference to Figure 1.

In the following, such candidate panel of genes corresponding to the second candidate panel of genes above disclosed with reference to Figure 1 will be called *"first new candidate panel".*

The computation above disclosed is repeated, on the same training set, for any other parameter vector present in the parameter space.

To summarize, in the training step 100, the input is a parameter space comprising, for example, 500 parameter vectors, and the sub-matrix of the master matrix comprising the 1000 patients of the training set with the initial 24,000 genes.

At the end of the training step 100, a coherent voting network has been construed for each parameter vector (therefore, for example a total of 500 coherent voting networks), each coherent voting network having a first new candidate panel associated to it (therefore, a total of 500 first new candidate panels).

All such first new candidate panels corresponding to all the parameter vectors in the parameter space are collected.

These first new candidate panels are then passed to the first filtering step 102, so as to provide to the next validation phase a limited number of candidate panels to consider further.

### 2.2 Validation phase

In the validation step 104 of Figure 2, the steps of the method of Figure 1 (with a few omitted steps explained here below) are applied, for a predetermined parameter vector of the parameter space as above disclosed, on the training set and on the validation set, and for one of the first new candidate panels of genes having passed the first filtering step 102 of Figure 2.

Then, all the steps are repeated for all the parameter vectors of the parameter space.

Then, all the steps are repeated, with all the other first new candidate panels of genes, for all the parameter vectors of the parameter space.

The validation phase uses the second set of 500 patients as follows: for each patient p in the validation set, in turns, a patient p is added to the training set with the survival information left undefined.

The vote of the corresponding voting network so constructed is taken as the prediction of the voting network for the patient p.

After a vote has been assigned to the patient p, the patient p is removed from the validation set and a next patient p of the validation set is processed as above disclosed.

The process is repeated for all the p patients of the validation set.

The survival data of the 500 patients of the validation set are not used in these bipartite graphs, and thus the prediction for them using the two-level voting schemes, candidate panel and parameter vector, is completely unbiased.

At the end of this validation phase, each patient of the validation set has been assigned to a class, survival or not survival, with reference to each first new candidate panel and parameter vector used for performing the method steps of the method of Figure 1.

The quality of the prediction is then measured by comparing the classes assigned to each of the 500 patients of the validation set, with reference to each different first new candidate panels, with the survival data associated to said 500 patients as contained in the METABRIC database.

These quality measurements are used in the second filtering step 106 of Figure 2 to select a small number of high quality gene panels, and associated parameter vectors.

Typically, the validation phase allows removing almost 95% of the first new candidate panels and parameter vectors, and the remaining 5% first new candidate panels and parameter vectors with the highest performances are sent to the last testing phase.

In the following, said remaining 5% first new candidate panels will be called *"second new candidate panels".*

### 2.3 Testing phase

In the testing step 108 of Figure 2, the steps of the method of Figure 1 (with a few omitted steps explained here below) are applied, for a predetermined associated parameter vector of the vectors obtained after the second filtering step 106, on the training set and on the testing set, for one of the second new candidate panels of genes having passed the second filtering of step 106 of Figure 2.

Then, all the steps are repeated for all the associated parameter vectors.

Then, all the steps are repeated, with all the other second new candidate panels of genes, for all the associated parameter vectors.

The testing phase uses the third set of 500 patients as follows: for each patient p in the testing set, in turns, a patient p is added to the training set with the survival information left undefined.

The vote of the corresponding voting network so constructed is taken as the prediction of the voting network for the patient p.

After a vote has been assigned to the patient p, the patient p is removed from the training set and a next patient p of the testing set is processed as above disclosed.

The survival data of the 500 patients of the training set are not used in these bipartite graphs, and thus the prediction for them using the two-level voting schemes, candidate panel and parameter vector, is completely unbiased.

At the end of this training phase, each patient of the testing set has been assigned to a class, survival or not survival, with reference to each candidate panels and parameter vector used for performing the method steps of the method of Figure 1.

The quality of the prediction is then measured by comparing the classes assigned to each of the 500 patients of the testing set, with reference to each different candidate panels, with the survival data associated to said 500 patients as contained in the METABRIC database.

These quality measurements are used in the third filtering step 110 of Figure 2 to select, among the second new candidate panels, one high quality gene panel (the final panel of biomarkers) and a final parameter vector.

### 2.4 Stability Analysis

The stability analysis step 112 is based on a leave-one-patient-out methodology, and it allows assessing the stability of the final parameter vector and of the final panel of biomarkers at the end of the third filtering step 110.

The stability is tested as follows.

The testing step 108 and the third filtering step 110 are repeated on the same second new candidate panels and parameter vectors as obtained at the end of the testing step 108, but masking one patient from the testing set.

This computation is repeated by unmasking the currently masked patient, and masking the next one.

This is repeated for all the patients of the testing set, and the best performing configuration (final panel, final parameter vector) is recorded in each case.

If the final panel of biomarkers and final parameter vector as obtained at the end of the third filtering step 110 occurs a majority of times as the best performing configuration with the masked patient, then this solution is termed *"stable"* with respect to perturbations of the testing set of patients.

### 3. Variants of the coherent voting network construction method between the training, validating and testing phases.

As above disclosed, the complete sequence of steps of the method of Figure 1 has been disclosed with reference to the training step 100.

When the method steps of Figure 1 are applied in the validation step 104, in the testing step 108 and in the stability analysis step 112, some steps are omitted.

The omitted steps are those whose purpose is to reduce the number of genes, since a candidate gene panel is supposed fixed when the method of Figure 1 is applied for validation, testing, or stability analysis.

Specifically step 2, steps 10, step 12 and step 14 are omitted.

The communities produced at the end of step 8 are passed directly to the quality assessment step (step 16).

In order to classify any new unknown patient, not belonging to the METABRIC dataset, the testing step 108 is reapplied with the selected panel of biomarkers and the selected parameter vector.

In particular, steps 1 to 20 (with the exception of steps 2, 10, 12 and 14 as above disclosed) of the method of Figure 1 are applied to a cohort of 1001 patients, namely the 1000 patients of training set plus the new unknown patient, to find an A or B class for her.

The present invention allows the clinician to predict whether the patient will survive more or less than five years.

The advantage for the patient is the possibility to personalize any therapeutic choices performed with the aid of the clinician in a further analysis, taking into consideration her molecular prognostic profile, with higher chance of an effective cure and survival.

The advantage for the clinician is to have a tool to validate base-line therapeutic choices (or suggest the need for alternatives). The advantage for the whole health system is a better discrimination among those patients requiring expensive and invasive cure (e.g. chemotherapy), and those that would benefit from less expensive and invasive ones (e.g. hormonal therapy).

Clearly, the principle of the invention remaining the same, the embodiments and the details of production can be varied considerably from what has been described and illustrated purely by way of non-limiting example, without departing from the scope of protection of the present invention as defined by the attached claims.

## Claims

1. Method for creating a coherent voting network useful in predicting a likelihood of long-term survival of breast cancer in a breast cancer patient, the method including the steps of:
a) organizing (1) predetermined data from a cohort of patients in a master matrix having in its row the list of patients, each having an associated survival or nonsurvival class representing whether the patient had survived or not after a tumor removal, and having in its column the expression value of a panel of genes;
b) applying (2) a predetermined statistical test to each gene of the master matrix to evaluate which genes better discriminates the survival or not-survival classes, thus obtaining a first candidate panel of genes;
c) discretizing (4) the expression value of each of the genes belonging to said first candidate panel in sub-intervals capable of discriminating the survival or not-survival classes, thus obtaining a quantized master matrix;
d) converting (6) the quantized master matrix in a first bipartite graph (G) comprising patient-nodes of both classes, and gene-nodes representatives of the sub-intervals of the expression value of genes of the first candidate panel;
e) applying (8) a predetermined algorithm to the bipartite graph (G) to obtain a collection of bipartite communities including both patient-nodes and gene-nodes, the collection of communities providing coverage of the nodes of the bipartite graph (G);
f) applying (10) a further predetermined algorithm to the communities obtained at the end of said step e), restricted to the gene-nodes only, thus obtaining a subset of the genes of the first candidate panel constituting a second candidate panel capable of reproducing the same structure of bipartite communities;
g) creating (12) an updated bipartite graph (G') comprising nodes-patients and nodes-gene whose genes belongs to the second candidate panel;
h) repeating (14) step e) on the updated bipartite graph (G') and checking if the communities obtained are similar to the ones obtained at step e);
i) applying (16), at each community, a decision function on each patient belonging to it, to determine whether to assign her at the survival or not-survival class, then checking which class the decision function has assigned to each patient, for each community, and assigning a final class to the patient based on a majority rule;
j) checking (18), for each patient belonging to the various communities of step i), whether the class assigned is the same as the class of the master matrix, thus obtaining coherent patients;
k) checking (20) whether the percentage of coherent patients in the voting network is greater than a predetermined threshold, thus obtaining a coherent voting network.

2. The method of claim 1, wherein applying (2) a predetermined statistical test comprises setting the value of a first group of parameters including the type of test performed, the maximum p-value for accepting a gene among those under test, and a threshold for accepting a fold change of the gene under test.

3. The method of claim 1 or 2, wherein discretizing (4) includes applying methods based on the information theory.

4. The method of any of the preceding claims, wherein discretizing (4) comprises setting the value of a second group of parameters including the specific objective function used for the determination of cut points, the minimum and maximum number of cut points generated, the minimum and maximum number of patients in each interval generated by a cut point, and the number of significant digits to be to be considered in the gene expression measurements.

5. The method of any of the preceding claims, wherein obtaining the bipartite graph (G) comprises using all the rows of the master matrix and only the columns of the master matrix whose values of the genes are the discretized ones.

6. The method of any of the preceding claims, wherein applying (8) a predetermined algorithm to the bipartite graph (G) comprises setting a density threshold on the graph (G).

7. The method of any of the preceding claims, wherein the decision function comprises "unanimity" or "majority".

## Patentansprüche

1. Verfahren zum Erzeugen eines kohärenten Abstimmungsnetzwerks, das bei der Vorhersage der Wahrscheinlichkeit des langfristigen Überlebens von Brustkrebs bei einem Brustkrebspatienten nützlich ist, wobei das Verfahren die folgenden Schritte umfasst:
a) Organisieren (1) vorbestimmter Daten aus einer Kohorte von Patienten in einer Hauptmatrix, die in ihrer Zeile die Liste von Patienten aufweist, von denen jeder eine zugeordnete Überlebens- oder Nicht-Überlebensklasse hat, die darstellt, ob der Patient nach einer Tumorentfernung überlebt hat oder nicht, und die in ihrer Spalte den Expressionswert einer Gruppe von Genen aufweist;
b) Anwenden (2) eines vorbestimmten statistischen Tests auf jedes Gen der Hauptmatrix, um zu bewerten, welche Gene die Überlebens- bzw. Nicht-Überlebensklassen besser unterscheiden, wodurch eine erste Kandidatengruppe von Genen erhalten wird;
c) Diskretisieren (4) des Expressionswerts jedes der Gene, die zu der ersten Kandidatengruppe gehören, in Unterintervalle, die in der Lage sind, die Überlebensoder Nicht-Überlebensklassen zu unterscheiden, wodurch eine quantisierte Hauptmatrix erhalten wird;
d) Umwandeln (6) der quantisierten Hauptmatrix in einen ersten zweiteiligen Grafen (G), der Patientenknoten beider Klassen und Genknoten umfasst, die die Unterintervalle des Expressionswerts von Genen der ersten Kandidatengruppe repräsentieren;
e) Anwenden (8) eines vorbestimmten Algorithmus auf den zweiteiligen Grafen (G), um eine Sammlung von zweiteiligen Gemeinschaften zu erhalten, die sowohl Patientenknoten als auch Genknoten enthalten, wobei die Sammlung von Gemeinschaften die Knoten des zweiteiligen Grafen (G) abdeckt;
f) Anwenden (10) eines weiteren vorbestimmten Algorithmus auf die am Ende des Schritts e) erhaltenen Gemeinschaften, beschränkt auf die Genknoten, wodurch eine Teilmenge der Gene der ersten Kandidatengruppe erhalten wird, die eine zweite Kandidatengruppe bilden, die in der Lage ist, dieselbe Struktur der zweistufigen Gemeinschaften zu reproduzieren;
g) Erstellen (12) eines aktualisierten zweistufigen Grafen (G'), der Knoten-Patienten und Knoten-Gene umfasst, deren Gene zur zweiten Kandidatengruppe gehören;
h) Wiederholen des (14) Schritts e) auf dem aktualisierten zweiteiligen Grafen (G') und Überprüfen, ob die erhaltenen Gemeinschaften denen aus Schritt e) ähnlich sind;
i) Anwenden (16), in jeder Gemeinschaft, einer Entscheidungsfunktion auf jeden Patienten, der zu ihr gehört, um zu bestimmen, ob er der Überlebens- oder der Nicht-Überlebensklasse zuzuordnen ist, dann Überprüfen, welche Klasse die Entscheidungsfunktion jedem Patienten für jede Gemeinschaft zugeordnet hat, und Zuordnen einer endgültigen Klasse zu dem Patienten auf der Grundlage einer Mehrheitsregel;
j) Prüfen (18) für jeden Patienten, der zu den verschiedenen Gemeinschaften aus Schritt i) gehört, ob die zugewiesene Klasse mit der Klasse der Hauptmatrix übereinstimmt, um so kohärente Patienten zu erhalten;
k) Prüfen (20), ob der Prozentsatz der kohärenten Patienten im Abstimmungsnetzwerk größer als ein vorbestimmter Schwellenwert ist, wodurch ein kohärentes Abstimmungsnetzwerk erhalten wird.

2. Verfahren nach Anspruch 1, wobei das Anwenden (2) eines vorbestimmten statistischen Tests das Einstellen des Werts einer ersten Gruppe von Parametern umfasst, einschließlich der Art des durchgeführten Tests, des maximalen p-Werts für die Akzeptanz eines Gens unter den zu testenden Genen und eines Schwellenwerts für die Akzeptanz einer Faltenänderung des zu testenden Gens.

3. Verfahren nach Anspruch 1 oder 2, wobei das Diskretisieren (4) das Anwenden von Verfahren umfasst, die auf der Informationstheorie basieren.

4. Verfahren nach einem der vorhergehenden Ansprüche, wobei das Diskretisieren (4) das Einstellen des Werts einer zweiten Gruppe von Parametern umfasst, einschließlich der spezifischen Zielfunktion, die für das Bestimmen von Schnittpunkten, der minimalen und maximalen Anzahl der erzeugten Schnittpunkte, der minimalen und maximalen Anzahl der Patienten in jedem durch einen Schnittpunkt erzeugten Intervall und der Anzahl signifikanter Stellen verwendet wird, die bei den Genexpressionsmessungen zu berücksichtigen sind.

5. Verfahren nach einem der vorhergehenden Ansprüche, wobei das Erhalten des zweiteiligen Grafen (G) das Verwenden aller Zeilen der Hauptmatrix und nur der Spalten der Hauptmatrix umfasst, deren Werte der Gene die diskretisierten sind.

6. Verfahren nach einem der vorhergehenden Ansprüche, wobei das Anwenden (8) eines vorbestimmten Algorithmus auf den zweiteiligen Grafen (G) das Festlegen eines Schwellenwerts für die Dichte des Grafen (G) umfasst.

7. Verfahren nach einem der vorhergehenden Ansprüche, wobei die Entscheidungsfunktion "Einstimmigkeit" oder "Mehrheit" umfasst.

## Revendications

1. Méthode de création d'un réseau de vote cohérent utile pour prédire la probabilité de survie à long terme d'un cancer du sein chez une patiente atteinte d'un cancer du sein, la méthode comprenant les étapes consistant à :
a) organiser (1) des données prédéterminées provenant d'une cohorte de patients dans une matrice maîtresse ayant pour ligne la liste des patients, chacun ayant une classe de survie ou de non-survie associée représentant le fait que le patient a survécu ou non après l'ablation d'une tumeur, et ayant pour colonne la valeur d'expression d'un panel de gènes ;
b) appliquer (2) un test statistique prédéterminé à chaque gène de la matrice principale afin d'évaluer quels sont les gènes qui discriminent le mieux les classes de survie ou de non-survie, obtenant ainsi un premier panel de gènes candidats ;
c) discrétiser (4) la valeur d'expression de chacun des gènes appartenant audit premier panel de candidats en sous-intervalles capables de discriminer les classes de survie ou de non-survie, obtenant ainsi une matrice maîtresse quantifiée ;
d) convertir (6) la matrice maîtresse quantifiée en un premier graphe bipartite (G) comprenant des noeuds-patients des deux classes et des noeuds-gènes représentatifs des sous-intervalles de la valeur d'expression des gènes du premier panel de candidats ;
e) appliquer (8) un algorithme prédéterminé au graphe bipartite (G) pour obtenir une collection de communautés bipartites comprenant à la fois des noeuds-patients et des noeuds-gènes, la collection de communautés assurant la couverture des noeuds du graphe bipartite (G) ;
f) appliquer (10) un autre algorithme prédéterminé aux communautés obtenues à l'issue de ladite étape e), en se limitant aux seuls noeuds-gènes, et obtenir ainsi un sous-ensemble de gènes du premier panel de candidats constituant un deuxième panel de candidats capable de reproduire la même structure de communautés bipartites ;
g) créer (12) un graphe bipartite actualisé (G') comprenant des noeuds-patients et des noeuds-gènes dont les gènes appartiennent au deuxième panel de candidats ;
h) répéter (14) l'étape e) sur le graphe bipartite actualisé (G') et vérifier si les communautés obtenues sont similaires à celles obtenues à l'étape e) ;
i) appliquer (16), à chaque communauté, une fonction de décision à chaque patient appartenant à cette communauté, afin de déterminer s'il faut l'affecter à la classe de survie ou de non-survie, puis vérifier quelle classe la fonction de décision a affectée à chaque patient, pour chaque communauté, et affecter une classe finale au patient sur la base d'une règle de majorité ;
j) vérifier (18), pour chaque patient appartenant aux différentes communautés de l'étape i), si la classe attribuée est la même que la classe de la matrice maîtresse, ce qui permet d'obtenir des patients cohérents ;
k) vérifier (20) si le pourcentage de patients cohérents dans le réseau de vote est supérieur à un seuil prédéterminé, ce qui permet d'obtenir un réseau de vote cohérent.

2. Méthode selon la revendication 1, dans laquelle l'application (2) d'un test statistique prédéterminé consiste à définir la valeur d'un premier groupe de paramètres comportant le type de test effectué, la valeur p maximale pour l'acceptation d'un gène parmi ceux testés, et un seuil pour l'acceptation d'un changement de pli du gène testé.

3. Méthode selon la revendication 1 ou 2, dans laquelle la discrétisation (4) comporte l'application de méthodes basées sur la théorie de l'information.

4. Méthode selon l'une quelconque des revendications précédentes, dans laquelle la discrétisation (4) consiste à définir la valeur d'un deuxième groupe de paramètres comportant la fonction objective spécifique utilisée pour la détermination des points de coupure, le nombre minimum et maximum de points de coupure générés, le nombre minimum et maximum de patients dans chaque intervalle généré par un point de coupure, et le nombre de chiffres significatifs à prendre en compte dans les mesures de l'expression génique.

5. Méthode selon l'une quelconque des revendications précédentes, dans laquelle l'obtention du graphe bipartite (G) consiste à utiliser toutes les lignes de la matrice maîtresse et uniquement les colonnes de la matrice maîtresse dont les valeurs des gènes sont celles qui ont été discrétisées.

6. Méthode selon l'une quelconque des revendications précédentes, dans laquelle l'application (8) d'un algorithme prédéterminé au graphe bipartite (G) comprend la définition d'un seuil de densité sur le graphe (G).

7. Méthode selon l'une quelconque des revendications précédentes, dans laquelle la fonction de décision comprend « l'unanimité » ou la « majorité ».
